# FASCICULE DE BREVET EUROPEEN

(11) **EP 0 897 298 B1**
(45) Date de publication et mention de la délivrance du brevet: **26.03.2003**
(21) Numéro de dépôt: 97906225.4
(22) Date de dépôt: 19.02.1997
(51) Int. Cl.: A61K 7/06, A61K 7/00

(54) **COMPOSITION COSMETIQUE DE FIXATION ET DE BRILLANCE EN AEROSOL ET PROCEDES**
KOSMETISCHE AEROSOLZUSAMMENSETZUNG FÜR HAARFESTIGER MIT GLANZ UND BEHANDLUNGSVERFAHREN
COSMETIC AEROSOL FIXATIVE COMPOSITION PROVIDING SHINE, AND METHODS

(30) Priorité: 22.02.1996 FR 9602206
(43) Date de publication de la demande: 24.02.1999
(73) Titulaire: L'OREAL, 75008 Paris (FR)
(72) Inventeur: LESAULNIER, Claire-Marie, F-75009 Paris (FR); STURLA, Jean-Michel, F-92100 Boulogne-Billancourt (FR)
(74) Mandataire: Miszputen, Laurent
(86) Numéro de dépôt international: FR9700308
(87) Numéro de publication internationale: WO97030681

(56) Documents cités:
- EP-A- 0 205 306
- WO-A-95/00108
- FR-A- 2 706 296
- HOUSEHOLD AND PERSONAL PRODUCTS INDUSTRY, vol. 20, no. 11, Novembre 1983, NEW JERSEY (USA), pages 49-58, XP002021427 R. JACKIE GRANT: "silicones for ethnic hair care"
- SOAP,COSMETICS,CHEMICAL SPECIALTIES, vol. 63, no. 10, Octobre 1987, NEW YORK (USA), XP002032118 C. M. HANDT: "hair fixatives"
- "International Cosmetic Ingredient Dictionary and Handbook2, 7ème édition, 1997, publié par "The Cosmetic, Toiletry, and Fragrance Association", page 1089

## Description

La présente invention a trait à une composition cosmétique pressurisée en aérosol pour le traitement des matières kératiniques, en particulier des cheveux, comprenant (a) un jus comprenant au moins un polymère fixant, au moins 5% en poids par rapport au poids total de la composition pressurisée d'une silicone arylée non volatile et (b) entre 15 et 70% en poids d'au moins un propulseur, ainsi qu'au procédé de traitement des matières kératiniques à l'aide de cette composition. Dans le domaine cosmétique, une composition pressurisée en aérosol comprend communément une composition appelé jus et un agent propulseur.

Les compositions de maintien ou de mise en forme des cheveux contenant dans leur formulation des polymères de coiffage (polymères fixants) présentent généralement l'inconvénient de rendre difficile le démêlage, le recoiffage ou le brossage des cheveux, en particulier pendant un brushing. Les polymères coiffants ont également tendance à rendre les cheveux ternes.

L'association de dérivés siliconés avec des polymères fixants est connue dans des compositions cosmétiques pour le maintien et/ou la fixation de la coiffure. Il a été constaté que ces dérivés siliconés améliorent les propriétés de démêlage, de douceur et de brillance des cheveux traités à l'aide de ces compositions. Cependant, d'une part, les dérivés siliconés ne sont pas favorables aux propriétés coiffantes des compositions contenant des polymères fixants et, d'autre part, les propriétés de brillance ne sont pas encore satisfaisantes.

Le document FR 2 706 296 décrit une composition cosmétique capillaire de brillance comprenant une silicone arylée et un éther de cellulose cationique. L'éther de cellulose cationique est un agent moussant. Le document WO95/00108 décrit une composition comprenant une association de deux polymères fixants. Le premier polymère fixant est un polymère greffé ayant un squelette vinylique et des greffons siliconés, ces greffons comprenant éventuellement des groupements aryles. Le deuxième polymère fixant est une résine non siliconée.

Il existe des produits dits "brillantant" qui s'appliquent en soin de finition, c'est à dire sur cheveux séchés. Ces produits s'appliquent difficilement car si la quantité appliquée est trop importante ou mal répartie, la chevelure a généralement un toucher et un aspect visuel gras. De plus, ces produits n'apportent aucune fixation.

Le but de la présente invention est donc de proposer des compositions permettant de fixer et/ou de mettre en forme la coiffure, ces compositions devant avoir de bonnes propriétés de tenue dans le temps et apporter d'excellentes propriétés de brillance.

Or, la demanderesse a découvert de manière surprenante qu'en utilisant des compositions contenant un polymère fixant en association avec au moins une silicone arylée non volatile et au moins un propulseur dans un solvant cosmétiquement acceptable, on obtient d'excellentes propriétés de brillance, un bon temps de séchage tout en ayant d'excellentes propriétés coiffantes et/ou fixantes.

La présente invention a donc pour objet une composition cosmétique préssurisée en aérosol comprenant, (a) un jus comprenant dans un milieu cosmétiquement acceptable au moins un polymère fixant, au moins une silicone arylée non volatile par rapport au poids du jus et (b) au moins un propulseur, la concentration en silicone arylée non volatile étant supérieure ou égale à 5% en poids par rapport au poids total de la composition.

De façon surprenante, la diminution du pouvoir fixant des compositions est limitée malgré la présence de quantité importante de silicone. Les propriétés coiffantes sont du même degré que celles d'une composition ne contenant que le polymère fixant. En particulier, le pouvoir fixant, la tenue dans le temps et le volume de chevelure sont très bons

Ces compositions ne poudrent pas sur la chevelure lors du brossage ou du peignage (il n'y a pas de pellicules blanches) et sont invisibles sur les cheveux. Les cheveux traités ont un toucher et un aspect visuel non gras.

De plus, le brossage et/ou le peignage de la chevelure après l'application du produit améliorent encore la brillance.

Dans le cadre de la présente demande, on entend par compositions cosmétiques pour le maintien de la coiffure toute composition ayant pour fonction de fixer temporairement la forme de la coiffure, comme par exemple les laques et sprays de coiffage. Par pouvoir fixant de la composition, on désigne l'aptitude de cette dernière à donner aux cheveux une cohésion de telle sorte que la mise en forme initiale de la coiffure soit conservée. Par polymère fixant, on entend tout polymère ayant pour fonction de fixer temporairement la forme de la coiffure.

Selon l'invention, on peut utiliser tout polymère fixant connu en soi. On peut utiliser en particulier un polymère fixant choisi parmi les polymères anioniques, cationiques, amphotères, non ioniques et leurs mélanges.
Les polymères fixants peuvent être utilisés sous forme solubilisée ou sous forme de dispersions de particules solides de polymère.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 et 3.000.000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 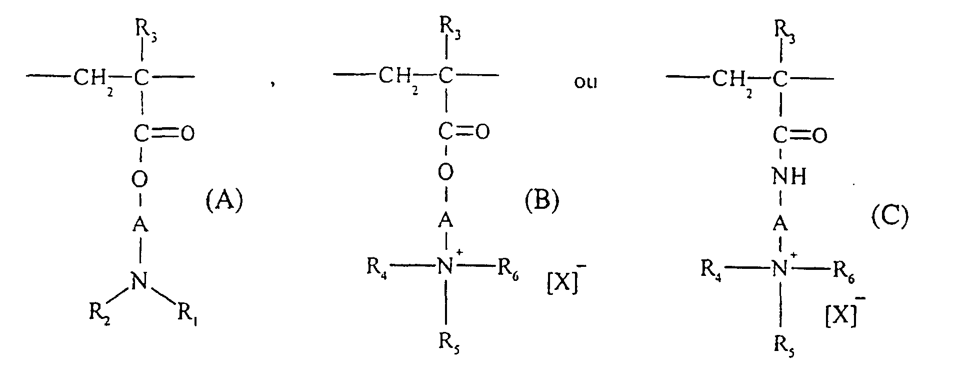 dans lesquelles:
   R₃ désigne un atome d'hydrogène ou un radical CH₃;
   A est un groupe alkyle linéaire ou ramifié de 1 à 6 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs monomères pouvant être choisis dans la famille des acrylamides, méthacrylamides, diacétones acrylamides, acrylamides et méthacrylamides substitués sur l'azote par des alkyles inférieurs en C₁-C₆, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymère méthacrylate de diméthyl amino éthyle/ vinylcaprolactame/vinylpyrrolidone tel que le produit vendu sous la dénomination GAFFIX VC 713 par la société ISP,
   - et le copolymère vinylpyrrolidone / méthacrylamide de diméthylaminopropyle quaternisé tel que le produit vendu sous la dénomination "GAFQUAT HS 100" par la société ISP.
(2) les polysaccharides quaternisés décrits plus particulièrement dans les brevets américains 3.589.578 et 4.031.307 tel que le produit commercialisé sous la dénomination JAGUAR C 13 S par la société MEYHALL;
(3) les copolymères quaternaires de vinylpyrrolidone et de vinylimidazole;
(4) les chitosanes ou leurs sels;
les sels utilisables sont en particulier les acétate, lactate, glutamate, gluconate ou le pyrrolidone carboxylate de chitosane .
Parmi ces composés, on peut citer le chitosane ayant un taux de désacétylation de 90,5% vendu sous la dénomination KYTAN BRUT STANDARD par la société ABER TECHNOLOGIES, le pyrrolidone carboxylate de chitosane vendu sous la dénomination KYTAMER PC par la société AMERCHOL.

Les polymères fixants anioniques généralement utilisés sont des polymères comportant des groupements dérivés d'acide carboxylique, sulfonique ou phosphorique et ont un poids moléculaire moyen en poids compris entre environ 500 et 5.000.000.
1) Les groupements carboxyliques sont apportés par des monomères mono ou diacides carboxyliques insaturés tels que ceux répondant à la formule : 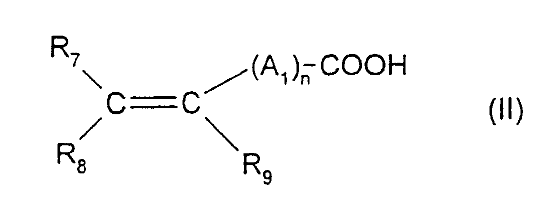 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur en C₁-C₆ ou carboxyle, R₉ désigne un atome d'hydrogène, un groupement alkyle inférieur en C₁-C₆, un groupement -CH₂-COOH, phényle ou benzyle ;
   Dans la formule précitée un radical alkyle inférieur désigne de préférence un groupement ayant 1 à 6 atomes de carbone et en particulier, méthyle et éthyle.
   Les polymères fixants anioniques à groupements carboxyliques préférés selon l'invention sont :
   A) les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
   B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copoiymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
   C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564. 110 et 2.439.798. Des produits commerciaux entrant dans cette classe sont les résines 28-29-30, 26-13-14 et 28-13-10 vendues par la société NATIONAL STARCH.
   D) les copolymères dérivés d'acides ou d'anhydrides carboxyliques monoinsaturés en C4-C8 choisis parmi :
      - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. ; De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
      - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
         les fonctions anhydrides de ces copolymères étant éventuellement monoestérifiées ou monoamidifiées.
      Ces polymères sont par exemple décrits dans les brevets français 2.350.384 et 2.357.241 de la demanderesse.
   E) les polyacrylamides comportant des groupements carboxylates.
2) Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire moyen en poids compris entre environ 1.000 et 100.000 ainsi que les copolymères avec un monomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire moyen en poids d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolyméres acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF et le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique /acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle /acide crotonique et les terpolymères acide crotonique / acétate de vinyle /néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;
B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène a,b-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléïque, fumarique ainsi que les monoesters d'alkyle ayant 1 à 4 atome de carbone des acides ou des anhydrides maléïque ou fumarique.
      Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de poyaminoamides de formule générale : 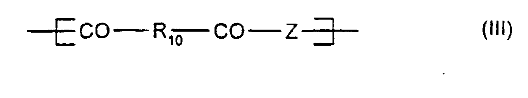 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 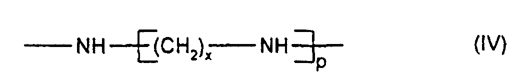 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 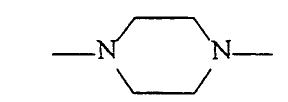
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule : 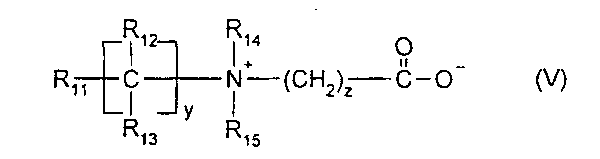 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl carboxyméthylammonio éthylméthacrylate de méthyle tel que le produit vendu sous la dénomination DIAFORMER Z301 par la société SANDOZ.
(5) les polymères dérivés du chitosane comportant des motifs monomères répondant aux formules suivantes : 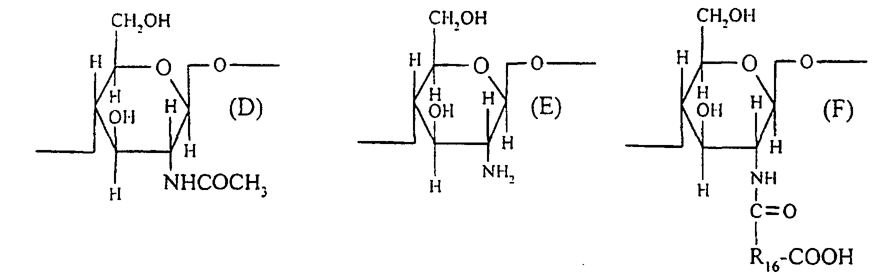 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 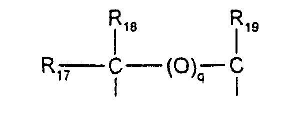 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, acoylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (VI) sont par exemple décrits dans le brevet français 1 400 366 : 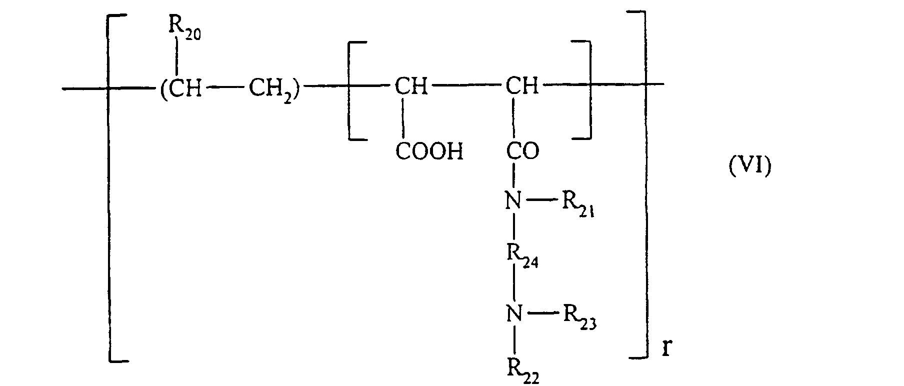 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant les significations mentionnées ci-dessus,
   ainsi que les homologues supérieurs de ces radicaux et contenant jusqu'à 6 atomes de carbone.
(8) Des polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VII)

      où D désigne un radical 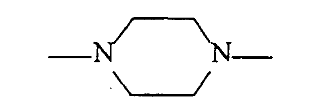 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VII')
   où D désigne un radical 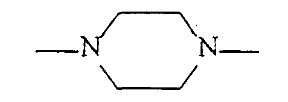 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi :
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les homopolymères de chlorure de vinyle tels que les produits proposés sous les noms de GEON 460X45, GEON 460X46 et GEON 577 par la société GOODRICH ;
- les cires de polyéthylène tels que les produits proposés sous les dénominations AQUACER 513 et AQUACER 533 par la société BYK CERA ;
- les cires de polyéthylène/polytétrafluoroéthylène tels que les produits proposés sous les dénominations DREWAX D-3750 par la société DREW AMEROID et WAX DISPERSION WD-1077 par la société R.T. NEWEY ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS
- les homopolymères de styrène tels que le produit RHODOPAS 5051 proposé par la société RHONE POULENC ;
- les copolymères de styrène et de (méth)acrylate d'alkyle tels que les produits MOWILITH LDM 6911, MOWILITH DM 611 et MOWILITH LDM 6070 proposés par la société HOECHST, les produits RHODOPAS SD 215 et RHODOPAS DS 910 proposés par la société RHONE POULENC;
- les copolymères de styrène, de méthacrylate d'alkyle et d'acrylate d'alkyle tels que le produit DAITISOL SPA proposé par la société WACKHERR ;
- les copolymères de styrène et de butadiène tels que les produits RHODOPAS SB 153 et RHODOPAS SB 012 proposés par la société RHONE POULENC ;
- les copolymères de styrène, de butadiène et de vinylpyridine tels que les produits GOODRITE SB VINYLPYRIDINE 2528X10 et GOODRITE SB VINYLPYRIDINE 2508 proposés par la société GOODRICH ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
Les radicaux alkyle des polymères non ioniques ont de 1 à 6 atomes de carbone sauf mention contraire.

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 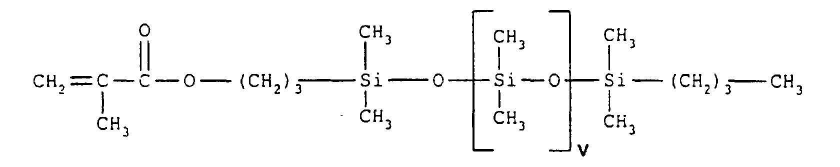 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon la présente invention, les polymères fixants sont de préférence des polymères anioniques.

Les polymères fixants anioniques ou amphotères peuvent être si nécessaire neutralisés partiellement ou totalement. Les agents de neutralisation sont par exemple la soude, la potasse, l'amino-2 méthyl-2 propanol-1, la monoéthanolamine, la triéthanolamine ou la triisopropanolamine, les acides minéraux ou organiques tels que l'acide chlorhydrique ou l'acide citrique.

Par silicone non volatile, on entend selon la présente invention, toute silicone présentant une pression de vapeur mesurée à 25°C à la pression atmosphérique (10⁵ Pa) de préférence inférieure à 0,01 mm Hg (2,6 Pa).

Les silicones arylées non volatiles comportent au moins un radical de type aryle éventuellement substitué. Les radicaux aryles sont par exemple phényle, naphtyle, benzyle ou phénéthyle.

Les silicones arylées non volatiles présentent préférentiellement la formule suivante (X): 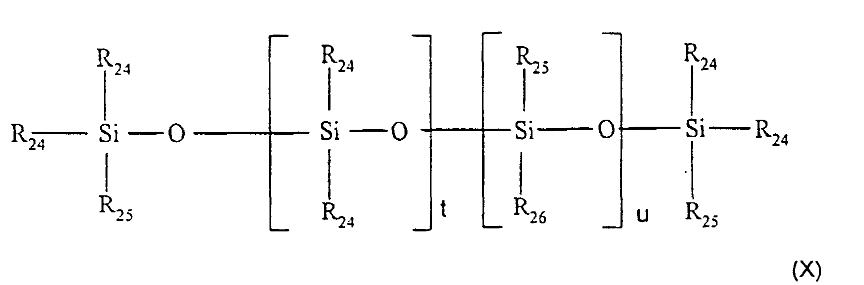 dans laquelle :
R₂₄, identique ou différent, désigne un radical alkyle en C₁-C₁₀.
R₂₆, identique ou différent, désigne un groupement aryle, ce groupement aryle pouvant comprendre un ou plusieurs cycles aryle, éventuellement substitués.
R₂₅ , identique ou différent, désigne R₂₆, R₂₄ ou Si(R₂₄)₃
t varie de 0 à 1000,
u varie de 1 à 1000,
la somme t+u peut varier de 1 à 2000.

Les substituants des groupements aryles peuvent être des groupes alkyles, alkényles, acyles, cétones, halogènes (par exemple Cl et Br), amines. Des exemples de groupements aryles sont le phényle, un groupement phényle substitué par des radicaux alkyle ou des radicaux alkényle en C₁-C₅ tel que allylphényle, méthylphényle, éthylphényle, vinylphényle et leurs mélanges.

De préférence , R₂₄ désigne un radical méthyle. De préférence , R₂₆ désigne un radical phényle.
De préférence , R₂₅ désigne un radical méthyle, phényle ou triméthylsilyle.
Plus particulièrement, la somme t+u varie de 1 à 1000.

Parmi les composés de formules (X), on peut utiliser par exemple la phényl triméthicone, la diphényl diméthicone ou la phényl diméthicone (dénominations INCI 5ème édition 1993).
A titre d'exemple de ces composés, on peut citer ceux vendus par la société BAYER sous le nom Huile BAYSILONE FLUID PD5, par la société DOW CORNING sous le nom DOW CORNING 556 FLUID, par RHONE POULENC sous les noms MIRASIL DPDM, RHODORSIL HUILE 510 V 100, RHODORSIL HUILE 550, RHODORSIL HUILE 510V500, RHODORSIL HUILE 710, sous les dénomination WACKER BELSIL PDM 20, PDM 200, PDM 1000 par la société WACKER.

Selon la présente invention, on utilise de préférence les silicones arylées non volatiles ayant un indice de réfraction supérieur à 1,46, et en particulier compris entre 1,48 et 1,7. L'indice de réfraction est mesuré à l'aide d'un réfractomètre selon des méthodes bien connues.

Le ou les polymères fixants sont par exemple présents dans des concentrations comprises entre 0,1% et 20% en poids, et de préférence dans des concentrations comprises entre 1% et 10% en poids, et plus particulièrement entre 2 et 8% en poids par rapport au poids total de la composition.

Le ou les polymères fixants sont par exemple présents dans des concentrations comprises entre 1% et 30% en poids, et de préférence dans des concentrations comprises entre 2% et 15% en poids, par rapport au poids total du jus.

La ou les silicones arylées non volatiles peuvent être présentes dans des concentrations comprises entre 5% et 40% en poids, et de préférence dans des concentrations comprises entre 7% et 30% en poids et encore plus particulièrement de 8 à 15% en poids par rapport au poids total de la composition pressurisée.

La ou les silicones arylées non volatiles peuvent être présentes dans des concentrations supérieures ou égales à 10% en poids, de préférence dans des concentrations comprises entre 10% et 30% en poids et encore plus particulièrement de 12 à 20% en poids par rapport au poids total du jus.

Le milieu cosmétiquement acceptable comprend généralement des solvants compatibles avec le polymère fixant et la silicone arylée non volatile. Ces solvants sont de préférence des alcools en C₁-C₆, qui peuvent être utilisés seuls ou en mélange.

Parmi ces alcools, on peut citer l'éthanol, l'isopropanol, les polyalcools tels que le diéthylèneglycol, les éthers de glycol, les monoalkyléthers de glycol, de diéthylèneglycol, de propylèneglycol ou de dipropylèneglycol. L'éthanol est particulièrement préféré.

Selon l'invention, les silicones arylées non volatiles sont de préférence solubilisées dans le jus.

Les compositions selon l'invention sont de préférence exemptes d'eau, c'est à dire qu'elle contiennent moins de 8% en poids d'eau par rapport au poids total de la composition et préférentiellement moins de 5%. Le séchage des compositions est ainsi plus rapide.

La concentration en alcool est en général comprise entre 10 et 90% en poids et de préférence entre 50 et 80% en poids par rapport au poids total de la composition pressurisée.

Le pH des compositions selon l'invention est généralement compris entre 2 et 9, et en particulier entre 3 et 8,5. Il peut être ajusté à la valeur choisie au moyen d'agents alcalinisants ou acidifiants habituellement utilisés en cosmétique pour ce type d'application.

La composition selon l'invention est pressurisée sous forme d'aérosol, l'aérosol comprend donc le jus (composition sans propulseur) et au moins un agent propulseur qui peut être choisi parmi les hydrocarbures volatils tels que le n-butane, le propane, l'isobutane, les hydrocarbures chlorés et/ou fluorés et leurs mélanges. On peut également utiliser en tant qu'agent propulseur le gaz carbonique, le protoxyde d'azote, le diméthyléther, l'azote, l'air comprimé et leurs mélanges.

Le jus et le propulseur peuvent se trouver dans le même compartiment ou dans des compartiments différents dans le récipient aérosol.

La composition de l'invention peut également contenir au moins un additif choisi parmi les épaississants, les esters d'acides gras, les esters d'acides gras et de glycérol, les silicones volatiles et les silicones non arylées, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Les compositions selon l'invention peuvent contenir des silicones volatiles. Ces silicones volatiles sont généralement présentes dans des concentrations comprises entre 0,5% et 40% en poids, et de préférence dans des concentrations comprises entre 1% et 25% en poids et encore plus particulièrement de 2 à 15% en poids par rapport au poids total de la composition pressurisée.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de lait, de crème, de lotion plus ou moins épaissie.

Les compositions selon l'invention peuvent être utilisées comme produits rincés et de préférence comme produits non-rincés notamment pour le maintien de la coiffure ou la mise en forme des matières kératiniques telles que les cheveux.

Elles sont plus particulièrement des produits de coiffage tels que des compositions de fixation (laques) et de coiffage.

L'invention a encore pour objet un procédé de traitement cosmétique des matières kératiniques telles que les cheveux consistant à appliquer sur celles-ci une composition telle que définie précédemment.

La préparation des compositions selon l'invention est réalisée selon des méthodes bien connues de l'état de la technique. En particulier, les ingrédients du jus sont mélangés puis conditionnés dans un récipient approprié selon l'utilisation envisagée. Le jus est alors placé dans un récipient qui est ensuite scellé par un système comportant une valve, le propulseur peut être alors introduit dans le récipient.

L'invention va être maintenant plus complètement illustrée à l'aide des exemples suivants qui ne sauraient être considérés comme la limitant aux modes de réalisation décrits. (Dans ce qui suit MA signifie Matière Active).

### EXEMPLE 1

On a préparé une composition de spray fixant conditionnée dans un récipient aérosol de composition suivante :
- Copolymère acétate de vinyle / acide crotonique vendu par la société BASF sous la dénomination LUVISET CA 66 2,5 g
- Amino-2 méthyl-2 propanol-1 qs neutralisation à 100% du polymère
- Silicone phénylée vendue sous la dénomination DC 556 par la société DOW CORNING 5 g
- Diméthyl éther 50 g
- Ethanol qsp 100 g

Cette composition est vaporisée sur des cheveux séchés, la coiffure reste en place. Les cheveux sont très brillants et ne présentent pas un toucher gras.

### EXEMPLE 2

On a préparé une composition de spray fixant conditionnée dans un récipient aérosol de composition suivante :
- Terpolymère acide crotonique / acétate de vinyle / néododécanoate de vinyle vendu sous la dénomination Résine 28-29-30 par la société NATIONAL STARCH 6 g
- Amino-2 méthyl-2 propanol-1 qs neutralisation à 100% du polymère
- Silicone phénylée vendue sous la dénomination DC 556 par la société DOW CORNING 12 g
- Diméthyl éther 60 g
- Ethanol qsp 100 g

La composition présente les mêmes propriétés que celles de l'exemple 1.

### EXEMPLE 3

On a préparé une composition de spray fixant conditionnée dans un récipient aérosol de composition suivante :
- Terpolymère acide acrylique / acrylate d'éthyle / n-tertio butylacrylamide vendu sous la dénomination ULTRAHOLD STRONG par la société BASF 4,9 g
- Amino-2 méthyl-2 propanol-1 qs neutralisation à 100% du polymère
- Phényl diméthicone vendu sous la dénomination BELSIL PDM 200 par la société WACKER 7 g
- Diméthyl éther 30 g
- Ethanol qsp 100 g

La composition présente les mêmes propriétés que celles de l'exemple 1.

### EXEMPLE 4

On a préparé une composition de spray fixant conditionnée dans un récipient aérosol de composition suivante :
- Copolymère méthylvinyléther/anhydride maléique mono estérifié vendu par la société ISP sous la dénomination GANTREZ ES 425 6 g
- Amino-2 méthyl-2 propanol-1 qs neutralisation à 20% du polymère
- Diphényldiméthicone vendu sous la dénomination MIRASIL DPDM par la société RHONE POULENC 15 g
- Diméthyl éther 50 g
- Ethanol qsp 100 g

La composition présente les mêmes propriétés que celles de l'exemple 1.

### EXEMPLE 5

On a préparé une composition de spray fixant conditionnée dans un récipient aérosol de composition suivante :
- Terpolymère acide acrylique / acrylate d'éthyle / n-tertio butylacrylamide vendu sous la dénomination ULTRAHOLD STRONG par la société BASF 7 g
- Amino-2 méthyl-2 propanol-1 qs neutralisation à 100% du polymère
- Silicone phénylée vendue sous la dénomination DC 556 par la société DOW CORNING 15 g
- Diméthyl éther 40 g
- Ethanol qsp 100 g

La composition présente les mêmes propriétés que celles de l'exemple 1.

## Revendications

1. Composition cosmétique pressurisée en aérosol, **caractérisée en ce qu'**elle comprend :
(a) un jus comprenant dans un milieu cosmétiquement acceptable au moins un polymère fixant, au moins une silicone arylée non volatile et
(b) au moins un propulseur,
la concentration en silicone arylée non volatile étant supérieure ou égale à 5% en poids par rapport au poids total de la composition et l'agent propulseur étant présent dans des concentrations comprises entre 15 et 70% en poids par rapport au poids total de la composition.

2. Composition selon la revendication précédente, **caractérisée en ce que** le ou les polymères fixants sont présents dans des concentrations comprises entre 0,1% et 20% en poids, et de préférence dans des concentrations comprises entre 1% et 10% en poids, et plus particulièrement entre 2 et 8% en poids par rapport au poids total de la composition.

3. Composition selon l'une quelconque des revendications précédentes, **caractérisée en ce que** la ou les silicones arylées non volatiles sont présentes dans des concentrations comprises entre 5% et 40% en poids et de préférence entre 7 et 30% en poids, par rapport au poids total de la composition.

4. Composition selon l'une quelconque des revendications précédentes, **caractérisée en ce que** la ou les silicones arylées non volatiles sont présentes dans des concentrations comprises entre 10% et 30% en poids et de préférence entre 12 et 20% en poids par rapport au poids total du jus.

5. Composition selon l'une quelconque des revendications précédentes, **caractérisée en ce que** le polymère fixant est choisi parmi les polymères anioniques, cationiques, amphotères, non ioniques et leurs mélanges.

6. Composition selon la revendication précédente, **caractérisée par le fait que** le polymère fixant anionique est choisi parmi :
- les polymères comportant des motifs carboxyliques dérivant de monomères mono ou diacides carboxyliques insaturés de formule : 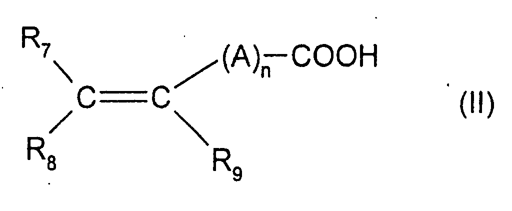
dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur en C₁-C₆ ou carboxyle, R₉ désigne un atome d'hydrogène, un groupement alkyle inférieur en C₁-C₆, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique.

7. Composition selon la revendication précédente, **caractérisée en ce que** le polymère fixant anionique est choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques ;
B) les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés ; les copolymères de ce type comportant dans leur chaîne un motif acrylamide éventuellement N-alkylé et/ou hydroxyalkylé, les copolymères d'acide acrylique et de méthacrylate d'alkyle en C₁-C₄ ;
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ;
D) les copolymères dérivés d'acides ou d'anhydrides carboxyliques monoinsaturés en C4-C8 choisis parmi :
- les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ;
- les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
les fonctions anhydrides de ces copolymères étant éventuellement monoestérifiées ou monoamidifiées.
E) les polyacrylamides comportant des groupements carboxylates.

8. Composition selon la revendication précédente, **caractérisée en ce que** le polymère fixant anionique est choisi parmi :
- les copolymères d'acide acrylique tels que le terpolymère acide acrylique/acrylate d'éthyle/N-tertiobutylacrylamide;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié ;
- les copolymères d'acide méthacrylique et de méthacrylate de méthyle ;
- le copolymère d'acide méthacrylique et d'acrylate d'éthyle;
- le copolymère acétate de vinyle/acide crotonique ;
- le terpolymère acétate de vinyle/acide crotonique/polyéthylèneglycol.

9. Composition selon la revendication 5, **caractérisée en ce que** le polymère fixant amphotère est choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

10. Composition selon la revendication précédente, **caractérisée en ce que** le polymère fixant amphotère est choisi parmi les copolymères dont la dénomination CTFA est Octylacrylamide/ acrylates/ butylaminoethylmethacrylate copolymer et les copolymères de méthacrylate de méthyle / diméthyl carboxyméthylammonio éthylméthacrylate de méthyle.

11. Composition selon la revendication 5, **caractérisé par le fait que** le polymère fixant non ionique est choisi parmi :
- les polyalkyloxazolines ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque ;
- les homopolymères de chlorure de vinyle ;
- les cires de polyéthylène ;
- les cires de polyéthylène/polytétrafluoroéthylène ;
- les copolymères de polyéthylène et d'anhydride maléïque ;
- les homopolymères d'acrylates d'alkyle et les homopolymères de méthacrylates d'alkyle ;
- les copolymères d'esters acryliques tels que par exemple les copolymères d'acrylates d'alkyle et de méthacrylates d'alkyles ;
- les copolymères d'acrylonitrile et d'un monomère non ionique choisi par exemple parmi le butadiène et les (méth)acrylates d'alkyle ;
- les homopolymères de styrène ;
- les copolymères de styrène et de (méth)acrylate d'alkyle ;
- les copolymères de styrène, de méthacrylate d'alkyle et d'acrylate d'alkyle ;
- les copolymères de styrène et de butadiène ;
- les copolymères de styrène, de butadiène et de vinylpyridine ;
- les copolymères d'acrylate d'alkyle et d'uréthanne.

12. Composition selon la revendication 5, **caractérisée en ce que** le polymère fixant cationique est choisi parmi :
- le copolymère d'acrylamide et de diméthylaminoéthyl méthacrylate quaternisé au sulfate de diméthyle,
- les copolymères d'acrylamide et de chlorure de méthacryloyloxyéthyltriméthylammonium,
- le copolymère d'acrylamide et de méthosulfate de méthacryloyloxyéthyltriméthylammonium,
- les copolymères vinylpyrrolidone / acrylate ou méthacrylate de dialkylaminoalkyle quaternisés ou non,
- les terpolymère méthacrylate de diméthyl amino éthyle/ vinylcaprolactame/vinylpyrrolidone,
- et le copolymère vinylpyrrolidone / méthacrylamide de diméthylaminopropyle quaternisé.

13. Composition selon l'une quelconque des revendications précédentes, **caractérisée en ce que** les silicones arylées non volatiles sont choisies parmi les silicones de formule suivante (X) : 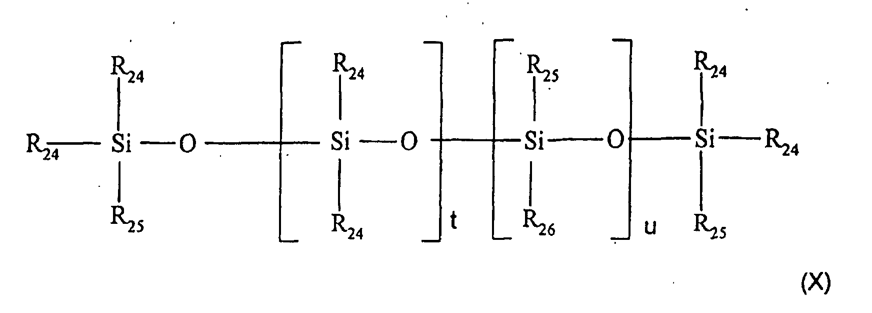 dans laquelle :
R₂₄, identique ou différent, désigne un radical alkyle en C₁-C₁₀.
R₂₆, identique ou différent, désigne un groupement aryle, ce groupement aryle pouvant comprendre un ou plusieurs cycles aryle, éventuellement substitués ;
R₂₅, identique ou différent, désigne R₂₆, R₂₄ ou Si(R₂₄)₃,
t varie de 0 à 1000,
u varie de 1 à 1000,
la somme t+u peut varier de 1 à 2000.

14. Composition selon la revendication précédente, **caractérisée en ce que** le groupement aryle est choisi parmi le phényle, un groupement phényle substitué par des radicaux alkyle ou des radicaux alkényle en C₁-C₅ et leurs mélanges.

15. Composition selon la revendication précédente, **caractérisée en ce que** la silicone arylée non volatile est choisie parmi la phényltriméthicone, la diphényl diméthicone et la phényl méthicone.

16. Composition selon l'une quelconque des revendications précédentes, **caractérisée en ce que** le milieu cosmétiquement acceptable comprend des alcools en C₁-C₆.

17. Composition selon l'une quelconque des revendications précédentes, **caractérisée en ce qu'**elle contient au moins une silicone volatile.

18. Composition selon la revendication précédente, **caractérisée en ce que** la ou les silicones volatiles sont présentes dans des concentrations comprises entre 0,5% et 40% en poids par rapport au poids total de la composition.

19. Composition selon l'une quelconque des revendications précédentes, **caractérisée par le fait que** l'agent propulseur est choisi parmi les hydrocarbures volatils tels que le n-butane, le propane, l'isobutane, les hydrocarbures chlorés et/ou fluorés, le gaz carbonique, le protoxyde d'azote, le diméthyléther, l'azote, l'air comprimé et leurs mélanges

20. Procédé de traitement cosmétique des matières kératiniques telles que les cheveux consistant à appliquer sur celles-ci une composition telle que définie à l'une quelconques des revendications précédentes.

## Claims

1. Cosmetic composition pressurized as an aerosol, **characterized in that** it comprises:
(a) a fluid comprising, in a cosmetically acceptable medium, at least one fixing polymer, at least one non-volatile arylsilicone, and
(b) at least one propellant,
the concentration of non-volatile arylsilicone being greater than or equal to 5% by weight relative to the total weight of the composition and the propellant being present in concentrations of between 15 and 70% by weight relative to the total wieght of the composition.

2. Composition according to the preceding claim, **characterized in that** the fixing polymer(s) is (are) present in concentrations of between 0.1% and 20% by weight, and preferably in concentrations of between 1% and 10% by weight, and more particularly between 2 and 8% by weight, relative to the total weight of the composition.

3. Composition according to either of the preceding claims, **characterized in that** the non-volatile arylsilicone(s) is (are) present in concentrations of between 5% and 40% by weight, and preferably between 7 and 30% by weight, relative to the total weight of the composition.

4. Composition according to any one of the preceding claims, **characterized in that** the non-volatile arylsilicone(s) is (are) present in concentrations of between 10% and 30% by weight, and preferably between 12 and 20% by weight, relative to the total weight of the fluid.

5. Composition according to any one of the preceding claims, **characterized in that** the fixing polymer is chosen from anionic, cationic, amphoteric and nonionic polymers, and mixtures thereof.

6. Composition according to the preceding claim, **characterized in that** the anionic fixing polymer is chosen from:
- polymers containing carboxylic units derived from unsaturated mono- or dicarboxylic acid monomers of formula: 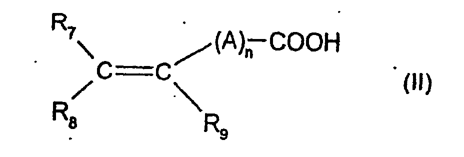 in which n is an integer from 0 to 10, A denotes a methylene group, optionally connected to the carbon atom of the unsaturated group, or to the neighbouring methylene group when n is greater than 1, via a hetero atom such as oxygen or sulphur, R₇ denotes a hydrogen atom or a phenyl or benzyl group, R₈ denotes a hydrogen atom or a lower C₁-C₆ alkyl or carboxyl group, R₉ denotes a hydrogen atom, a lower C₁-C₆ alkyl group or a -CH₂-COOH, phenyl or benzyl group;
- polymers comprising units derived from sulphonic acid, such as vinylsulphonic, styrenesulphonic or acrylamidoalkylsulphonic units.

7. Composition according to the preceding claim, **characterized in that** the anionic fixing polymer is chosen from:
A) acrylic or methacrylic acid homo- or copolymers, or salts thereof, copolymers of acrylic acid and of acrylamide, and salts thereof, sodium salts of polyhydroxycarboxylic acids;
B) copolymers of acrylic or methacrylic acids with a monoethylenic monomer such as ethylene, styrene, vinyl esters, acrylic or methacrylic acid esters, optionally grafted onto a polyalkylene glycol such as polyethylene glycol and optionally crosslinked; copolymers of this type containing an optionally N-alkylated and/or hydroxyalkylated acrylamide unit in their chain, copolymers of acrylic acid and of C₁-C₄ alkyl methacrylate;
C) copolymers derived from crotonic acid such as those containing vinyl acetate or propionate units in their chain and optionally other monomers such as allylic esters or methallylic esters, vinyl ether or vinyl ester of a linear or branched saturated carboxylic acid with a long hydrocarbon chain such as those containing at least 5 carbon atoms, it being possible for these polymers optionally to be grafted and crosslinked;
D) copolymers derived from C4-C8 monounsaturated carboxylic acids or anhydrides chosen from:
- copolymers comprising (i) one or more maleic, fumaric or itaconic acids or anhydrides and (ii) at least one monomer chosen from vinyl esters, vinyl ethers, vinyl halides, phenylvinyl derivatives, acrylic acid and its esters, the anhydride functions of these copolymers optionally being monoesterified or monoamidated;
- copolymers comprising (i) one or more maleic, citraconic or itaconic anhydrides and (ii) one or more monomers chosen from allylic or methallylic esters optionally containing one or more acrylamide, methacrylamide, α-olefin, acrylic or methacrylic ester, acrylic or methacrylic acid or vinylpyrrolidone group in their chain,
the anhydride functions of these copolymers optionally being monoesterified or monoamidated;
E) polyacrylamides containing carboxylate groups.

8. Composition according to the preceding claim, **characterized in that** the anionic fixing polymer is chosen from:
- acrylic acid copolymers, such as the acrylic acid/ethyl acrylate/N-tert-butylacrylamide terpolymer;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anhydrides with vinyl esters, vinyl ethers, vinyl halides, phenylvinyl derivatives and acrylic acid and esters thereof, such as methyl vinyl ether/monoesterified maleic anhydride copolymers;
- copolymers of methacrylic acid and of methyl methacrylate;
- the copolymer of methacrylic acid and of ethyl acrylate;
- the vinyl acetate/crotonic acid copolymer;
- the vinyl acetate/crotonic acid/polyethylene glycol terpolymer.

9. Composition according to Claim 5, **characterized in that** the amphoteric fixing polymer is chosen from polymers containing units derived from:
a) at least one monomer chosen from acrylamides or methacrylamides substituted on the nitrogen with an alkyl radical,
b) at least one acid comonomer containing one or more reactive carboxylic groups, and
c) at least one basic comonomer, such as esters containing primary, secondary, tertiary and quaternary amine substituents of acrylic and methacrylic acids, and the product of quaternization of dimethylaminoethyl methacrylate with dimethyl or diethyl sulphate.

10. Composition according to the preceding claim, **characterized in that** the amphoteric fixing polymer is chosen from the copolymers whose CTFA name is octylacrylamide/acrylates/butylaminoethyl methacrylate copolymer and the methyl methacrylate/methyl dimethylcarboxymethylammonioethyl methacrylate copolymers.

11. Composition according to Claim 5, **characterized in that** the nonionic fixing polymer is chosen from:
- polyalkyloxazolines;
- vinyl acetate homopolymers;
- copolymers of vinyl acetate and of acrylic ester;
- copolymers of vinyl acetate and of ethylene;
- copolymers of vinyl acetate and of maleic ester;
- vinyl chloride homopolymers;
- polyethylene waxes;
- polyethylene waxes/polytetrafluoroethylene;
- copolymers of polyethylene and of maleic anhydride;
- alkyl acrylate homopolymers and alkyl methacrylate homopolymers;
- acrylic ester copolymers, such as, for example, copolymers of alkyl acrylates and of alkyl methacrylates;
- copolymers of acrylonitrile and of a nonionic monomer chosen, for example, from butadiene and alkyl (meth)acrylates;
- styrene homopolymers;
- copolymers of styrene and of alkyl (meth)acrylate;
- copolymers of styrene, of alkyl methacrylate and of alkyl acrylate;
- copolymers of styrene and of butadiene;
- copolymers of styrene, of butadiene and of vinylpyridine;
- copolymers of alkyl acrylate and of urethane.

12. Composition according to Claim 5, **characterized in that** the cationic fixing polymer is chosen from:
- the copolymer of acrylamide and of dimethylaminoethyl methacrylate quaternized with dimethyl sulphate,
- copolymers of acrylamide and of methacryloyloxyethyltrimethylammonium chloride,
- the copolymer of acrylamide and of methacryloyloxyethyltrimethylammonium methosulphate,
- quaternized or non-quaternized vinylpyrrolidone/dialkylaminoalkyl acrylate or methacrylate copolymers,
- dimethylaminoethyl methacrylate/vinylcaprolactam/vinylpyrrolidone terpolymers, and
- the quaternized -vinylpyrrolidone/dimethylaminopropyl methacrylamide copolymer.

13. Composition according to any one of the preceding claims, **characterized in that** the non-volatile arylsilicones are chosen from the silicones of the following formula (X): 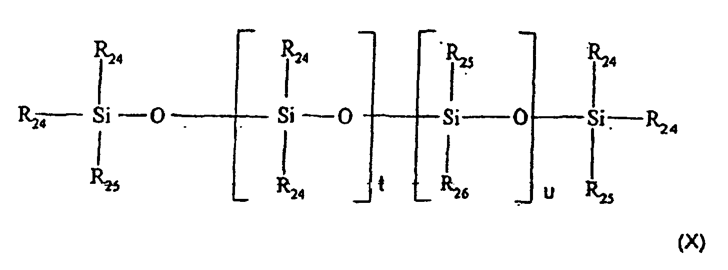 in which:
R₂₄, which may be identical or different, denotes a C₁-C₁₀ alkyl radical,
R₂₆, which may be identical or different, denotes an aryl group, it being possible for this aryl group to comprise one or more optionally substituted aryl rings;
R₂₅, which may be identical or different, denotes R₂₆, R₂₄ or Si(R₂₄)₃,
t ranges from 0 to 1000,
u ranges from 1 to 1000,
the sum t+u can range from 1 to 2000.

14. Composition according to the preceding claim, **characterized in that** the aryl group is chosen from phenyl and a phenyl group substituted with C₁-C₅ alkyl or alkenyl radicals, and mixtures thereof.

15. Composition according to the preceding claim, **characterized in that** the non-volatile arylsilicone is chosen from phenyltrimethicone, diphenyldimethicone and phenylmethicone.

16. Composition according to any one of the preceding claims, **characterized in that** the cosmetically acceptable medium comprises C₁-C₆ alcohols.

17. Composition according to any one of the preceding claims, **characterized in that** it contains at least one volatile silicone.

18. Composition according to the preceding claim, **characterized in that** the volatile silicone(s) is (are) present in concentrations of between 0.5% and 40% by weight relative to the total weight of the composition.

19. Composition according to any one of the preceding claims, **characterized in that** the propellant is chosen from volatile hydrocarbons such as n-butane, propane and isobutane, chlorohydrocarbons and/or fluorohydrocarbons, carbon dioxide, nitrous oxide, dimethyl ether, nitrogen and compressed air, and mixtures thereof.

20. Cosmetic treatment process for keratinous material such as the hair, which consists in applying a composition as defined in any one of the preceding claims to the hair.

## Patentansprüche

1. Unter Druck stehende kosmetische Aerosol-Zusammensetzung, **dadurch gekennzeichnet, daß** sie enthält:
(a) eine Flüssigkeit, die in einem kosmetisch akzeptablen Medium mindestens ein fixierendes Polymer und mindestens ein nichtflüchtiges aryliertes Silicon enthält, und
(b) mindestens ein Treibmittel,
wobei die Konzentration des nichtflüchtigen arylierten Silicons 5 Gew.-% oder mehr, bezogen auf das Gesamtgewicht der Zusammensetzung, beträgt und das Treibmittel in Konzentrationen von 15 bis 70 Gew.-%, bezogen auf das Gesamtgewicht der Zusammensetzung, vorliegt.

2. Zusammensetzung nach dem vorhergehenden Anspruch, **dadurch gekennzeichnet, daß** das oder die fixierenden Polymeren in Konzentrationen von 0,1 bis 20 Gew.-% und vorzugsweise in Konzentrationen von 1 bis 10 Gew.-% und insbesondere von 2 bis 8 Gew.-%, bezogen auf das Gesamtgewicht der Zusammensetzung, vorliegen.

3. Zusammensetzung nach einem der vorhergehenden Ansprüche, **dadurch gekennzeichnet, daß** das oder die nichtflüchtigen arylierten Silicone in Konzentrationen von 5 bis 40 Gew.-% und vorzugsweise von 7 bis 30 Gew.-%, bezogen auf das Gesamtgewicht der Zusammensetzung, vorliegen.

4. Zusammensetzung nach einem der vorhergehenden Ansprüche, **dadurch gekennzeichnet, daß** das oder die nichtflüchtigen arylierten Silicone in Konzentrationen von 10 bis 30 Gew.-% und vorzugsweise von 12 bis 20 Gew.-%, bezogen auf das Gesamtgewicht der Flüssigkeit, vorliegen.

5. Zusammensetzung nach einem der vorhergehenden Ansprüche, **dadurch gekennzeichnet, daß** das fixierende Polymer unter anionischen Polymeren, kationischen Polymeren, amphoteren Polymeren und nichtionischen Polymeren sowie ihren Gemischen ausgewählt ist.

6. Zusammensetzung nach dem vorhergehenden Anspruch, **dadurch gekennzeichnet, daß** das anionische fixierende Polymer ausgewählt ist unter:
- Polymeren mit Carboxylgruppen enthaltenden Einheiten, die von ungesättigten Mono- oder Dicarbonsäuremonomeren der Formel (II) 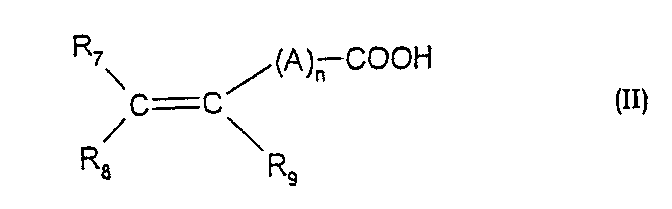 abgeleitet sind, in der bedeuten:
n eine ganze Zahl von 0 bis 10,
A eine Methylengruppe, die ggfs. am Kohlenstoffatom der ungesättigten Gruppe oder an der benachbarten Methylengruppe über ein Heteroatom wie Sauerstoff oder Schwefel gebunden ist, wenn n größer als 1 ist,
R₇ ein Wasserstoffatom, eine Phenylgruppe oder Benzyl,
R₈ ein Wasserstoffatom, eine niedere C₁₋₆-Alkylgruppe oder Carboxyl
und
R₉ ein Wasserstoffatom, eine niedere C₁₋₆-Alkylgruppe, eine Gruppe -CH₂-COOH, Phenyl oder Benzyl;
- Polymeren mit von Sulfonsäure abgeleiteten Einheiten, wie Vinylsulfonsäure-, Styrolsulfonsäure- und Acrylamidoalkylsulfonsäure-Einheiten.

7. Zusammensetzung nach dem vorhergehenden Anspruch, **dadurch gekennzeichnet, daß** das anionische fixierende Polymer ausgewählt ist unter:
A) Homo- und Copolymeren von Acrylsäure oder Methacrylsäure oder ihren Salzen, Copolymeren von Acrylsäure und Acrylamid und ihren Salzen und den Natriumsalzen von Polyhydroxycarbonsäuren;
B) Copolymeren von Acrylsäure oder Methacrylsäure mit einem monoethylenischen Monomer wie Ethylen, Styrol, Vinylestern, Acrylsäure- oder Methacrylsäureestern, die ggfs. auf einem Polyalkylenglykol wie Polyethylenglykol aufgepropft und ggfs. vernetzt sind; Copolymeren dieses Typs, die in ihrer Kette eine Acrylamidgruppe aufweisen, die gegebenenfalls N-alkyliert und/oder hydroxyalkyliert ist; Copolymeren von Acrylsäure und Alkylmethacrylat mit C₁₋₄-Alkylgruppen;
C) Copolymeren, die von Crotonsäure abgeleitet sind, wie z.B. Copolymeren, die in ihrer Kette Vinylacetat- oder Vinylpropionat-Einheiten und ggfs. weitere Monomere aufweisen, wie Allylester oder Methallylester, Vinylether oder Vinylester einer gesättigten geradkettigen oder verzweigten langkettigen Carbonsäure mit langer Kohlenwasserstoffkette, wie Carbonsäuren mit mindestens 5 Kohlenstoffatomen, wobei diese Polymeren ggfs. gepfropft und vernetzt sind;
D) Copolymeren, die von einfach ungesättigten C₄₋₈-Carbonsäuren oder ihren Anhydriden abgeleitet und ausgewählt sind unter:
- Copolymeren, die (i) eine oder mehrere unter Maleinsäure, Fumarsäure und Itaconsäure ausgewählte Säuren oder entsprechende Anhydride und (ii) mindestens ein Monomer enthalten, das unter den Vinylestern, Vinylethern, Vinylhalogeniden, Phenylvinylderivaten und Acrylsäure und ihren Estern ausgewählt ist,
wobei die Anhydridfunktionen dieser Copolymeren ggfs. monoverestert oder monoamidiert sind;
- Copolymeren, die (i) ein oder mehrere Anhydride von Maleinsäure, Citraconsäure oder Itaconsäure und (ii) ein oder mehrere Monomere enthalten, die unter den Allylestern oder Methallylestern ausgewählt sind und die ggfs. eine oder mehrere Gruppen von Acrylamid, Methacrylamid, α-Olefinen, Acrylestern oder Methacrylestern, Acrylsäure oder Methacrylsäure oder Vinylpyrrolidon in ihrer Kette enthalten,
wobei die Anhydridfunktionen dieser Copolymeren ggfs. monoverestert oder monoamidiert sind;
E) Polyacrylamiden, die Carboxylatgruppen enthalten.

8. Zusammensetzung nach dem vorhergehenden Anspruch, **dadurch gekennzeichnet, daß** das anionische fixierende Polymer ausgewählt ist unter:
- Copolymeren von Acrylsäure, wie das Acrylsäure/Ethylacrylat/N-t-Butylacrylamid-Terpolymer;
- Copolymeren, die von Crotonsäure abgeleitet sind, wie die Vinylacetat/Vinyl-t-butylbenzoat/ Crotonsäure-Copolymeren und die Crotonsäure/Vinylacetat/Vinylneododecanoat-Terpolymeren;
- Copolymeren von Maleinsäure, Fumarsäure, Itaconsäure oder ihren Anhydriden mit Vinylestern, Vinylethern, Vinylhalogeniden, Phenylvinylderivaten und Acrylsäure und ihren Estern, wie Methylvinylether/monoverestertes Maleinsäureanhydrid-Copolymere;
- Copolymeren von Methacrylsäure und Methylmethacrylat;
- Copolymeren von Methacrylsäure und Ethylacrylat;
- dem Vinylacetat/ Crotonsäure-Copolymer;
- dem Vinylacetat/Crotonsäure/Polyethylenglykol-Terpolymer.

9. Zusammensetzung nach Anspruch 5, **dadurch gekennzeichnet, daß** das amphotere fixierende Polymer unter Polymeren mit Einheiten ausgewählt ist, die abgeleitet sind von:
a) mindestens einem Monomer, das unter den am Stickstoffalkylsubstituierten Acrylamiden oder Methacrylamiden ausgewählt ist,
b) mindestens einem sauren Comonomer, das eine oder mehrere reaktive Carboxylgruppen enthält, und
c) mindestens einem basischen Comonomer, wie Estern von Acrylsäure und Methacrylsäure mit primären, sekundären, tertiären und quaternären Aminosubstituenten, und dem Quaternisierungsprodukt von Dimethylaminoethylmethacrylat mit Dimethylsulfat oder Diethylsulfat.

10. Zusammensetzung nach dem vorhergehenden Anspruch, **dadurch gekennzeichnet, daß** das amphotere fixierende Polymer ausgewählt ist unter Copolymeren mit der CTFA-Bezeichnung Octylacrylamide/acrylates/butylaminoethylmethacrylate copolymer und den Methylmethacrylat/Dimethylcarboxymethylammonioethylmethacrylat-Copolymeren.

11. Zusammensetzung nach Anspruch 5, **dadurch gekennzeichnet, daß** das nichtionische fixierende Polymer ausgewählt ist unter:
- den Polyalkyloxazolinen;
- den Vinylacetat-Homopolymeren;
- den Copolymeren von Vinylacetat und Acrylsäure;
- den Copolymeren von Vinylacetat und Ethylen;
- den Copolymeren von Vinylacetat und Maleinsäureester;
- den Vinylchlorid-Homopolymeren;
- den Polyethylenwachsen;
- den Polyethylen/Polytetrafluorethylen-Wachsen;
- den Copolymeren von Polyethylen und Maleinsäureanhydrid;
- den Alkylacrylat-Homopolymeren und den Alkylmethacrylat-Homopolymeren;
- den Copolymeren von Acrylsäureestern, wie z.B. den Copolymeren von Alkylacrylaten und Alkylmethacrylaten;
- den Copolymeren von Acrylnitril und einem nichtionischen Monomer, das beispielsweise unter Butadien und den Alkyl(meth)acrylaten ausgewählt ist;
- den Styrol-Homopolymeren;
- den Copolymeren von Styrol und Alkyl(meth)acrylat;
- den Copolymeren von Styrol, Alkylmethacrylat und Alkylacrylat;
- den Copolymeren von Styrol und Butadien;
- den Copolymeren von Styrol, Butadien und Vinylpyridin;
- den Copolymeren von Alkylacrylat und Urethan.

12. Zusammensetzung nach Anspruch 5, **dadurch gekennzeichnet, daß** das kationische fixierende Polymer ausgewählt ist unter:
- dem mit Dimethylsulfat quaternisierten Copolymer von Acrylamid und Dimethylaminoethylmethacrylat,
- den Copolymeren von Acrylamid und Methacryloyloxyethyltrimethylammoniumchlorid,
- dem Copolymer von Acrylamid und Methacryloyloxyethyltrimethylammonium-methosulfat,
- den Vinylpyrrolidon-dialkylaminoalkylacrylat oder -methacrylat-Copolymeren, die ggfs. quaternisiert sind,
- den Dimethylaminoethylmethacrylat/Vinylcaprolactam/Vinylpyrrolidon-Terpolymeren
und
- dem quaternisierten Vinylpyrrolidon/Dimethylamino-propylmethacrylamid-Copolymer.

13. Zusammensetzung nach einem der vorhergehenden Ansprüche, **dadurch gekennzeichnet, daß** die nichtflüchtigen arylierten Silicone unter den Siliconen der folgenden Formel (X) ausgewählt sind: 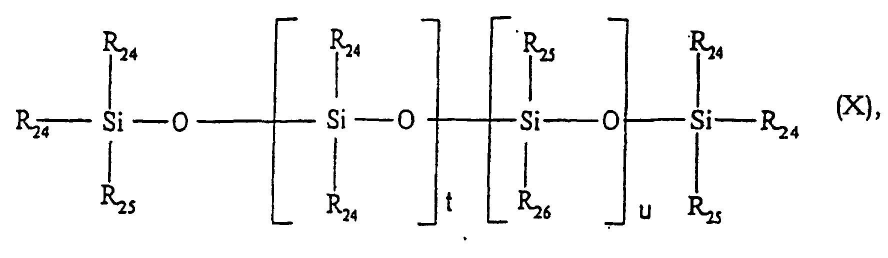 in der bedeuten:
R₂₄, die gleich oder verschieden sein können, C₁₋₁₀-Alkyl,
R₂₆, die gleich oder verschieden sein können, eine Arylgruppe, die einen oder mehrere Arylringe aufweisen kann, die ggfs. substituiert sind,
R₂₅, die gleich oder verschieden sein können, dasselbe wie R₂₆ oder R₂₄ oder Si(R₂₄)₃,
t 0 bis 1000
und
u 1 bis 1000,
wobei die Summe t+u 1 bis 2000 beträgt.

14. Zusammensetzung nach dem vorhergehenden Anspruch, **dadurch gekennzeichnet, daß** die Arylgruppe ausgewählt ist unter Phenyl, einer mit C₁₋₅-Alkylgruppen oder -alkenylgruppen substituierten Phenylgruppe und deren Gemischen.

15. Zusammensetzung nach dem vorhergehenden Anspruch, **dadurch gekennzeichnet, daß** das nichtflüchtige arylierte Silicon unter Phenyltrimethicon, Diphenyldimethicon und Phenylmethicon ausgewählt ist.

16. Zusammensetzung nach einem der vorhergehenden Ansprüche, **dadurch gekennzeichnet, daß** das kosmetisch akzeptable Medium C₁₋₆-Alkohole enthält.

17. Zusammensetzung nach einem der vorhergehenden Ansprüche, **dadurch gekennzeichnet, daß** sie mindestens ein flüchtiges Silicon enthält.

18. Zusammensetzung nach dem vorhergehenden Anspruch, **dadurch gekennzeichnet, daß** das oder die flüchtigen Silicone in Konzentrationen von 0,5 bis 40 Gew.-%, bezogen auf das Gesamtgewicht der Zusammensetzung, vorliegen.

19. Zusammensetzung nach einem der vorhergehenden Ansprüche, **dadurch gekennzeichnet, daß** das Treibmittel ausgewählt ist unter flüchtigen Kohlenwasserstoffen, wie n-Butan, Propan, Isobutan, chlorierten und/oder fluorierten Kohlenwasserstoffen, Kohlendioxidgas, Distickstoffmonoxid, Dimethylether, Stickstoff und Preßluft sowie ihren Gemischen.

20. Verfahren zur kosmetischen Behandlung von Keratinmaterialien wie Haaren, das darin besteht, eine Zusammensetzung wie in einem der vorhergehenden Ansprüchen definiert auf die Haare aufzubringen.
